# DEMANDE DE BREVET EUROPEEN

(11) **EP 0 770 365 A2**
(43) Date de publication de la demande: **02.05.1997**
(21) Numéro de dépôt: 96402243.8
(22) Date de dépôt: 22.10.1996
(51) Int. Cl.: A61F 2/04, A61F 2/06

(54) **Prothèse urétrale et son applicateur associé**

(30) Priorité: 23.10.1995 FR 9512426; 19.07.1996 FR 9609102
(71) Demandeur: BERBERIAN, Jean-Pierre, Martial, F-75004 Paris (FR)
(72) Inventeur: BERBERIAN, Jean-Pierre, Martial, F-75004 Paris (FR)
(74) Mandataire: Peuscet, Jacques

(57) **Abrégé**

L'invention concerne une prothèse urétrale (14) constituée par un élément souple réalisé en un matériau élastique biologiquement acceptable, ayant une longueur sensiblement égale à celle de la partie d'urètre à traiter, ledit élément constituant, lorsqu'il est en place dans l'urètre, une gouttière ayant une ouverture longitudinale entre deux bords longitudinaux mais étant mis en place dans l'urètre avec ses deux bords longitudinaux rapprochés pour constituer un tube dont le diamètre externe est voisin du diamètre interne de la zone d'urètre concernée, ladite gouttière étant maintenue en place dans l'urètre par la réaction élastique propre de sa paroi, qui, après mise en place dans l'urètre, tend à reprendre sa conformation d'origine en rétablissant l'ouverture longitudinale supprimée pour la mise en place, caractérisée par le fait qu'elle est constituée d'une feuille de matériau qui, avant sa conformation sous forme de tube cylindrique pour la mise en place dans l'urètre, a, à l'état libre, une courbure nulle ou inverse de celle qui lui est imposée pour constituer ledit tube.

## Description

L'invention concerne une prothèse urétrale et l'applicateur qui permet sa mise en place dans l'urètre d'un patient.

Les problèmes inhérents à la mise en oeuvre de ce type de prothèse résident en une bonne tolérance par le patient après l'implantation de la prothèse et en une absence de tout effet secondaire indésirable à long terme. L'urètre est un conduit relativement élastique mesurant environ 35-40 mm de long pour un diamètre de 4-7 mm chez la femme et 150-220 mm de long pour un diamètre de 7-12 mm chez l'homme (pendant la miction). L'urètre fait communiquer la vessie avec l'extérieur, le canal urinaire débouchant à l'extérieur par le méat du gland. A partir de la vessie, l'urètre comporte d'abord l'urètre intraprostatique, disposé entre le sphincter lisse et le sphincter strié, l'urètre intraprostatique passant entre les adénomes prostatiques. A la suite de l'urètre intraprostatique se trouve l'urètre pénien, qui comprend l'urètre pénien antérieur et l'urètre pénien postérieur, ce dernier étant lui-même divisible entre un urètre membraneux et l'urètre bulbaire, l'urètre membraneux se trouvant du côté du sphincter strié et l'urètre bulbaire se trouvant entre l'urètre membraneux et l'urètre pénien antérieur. Une prothèse selon l'invention peut être ou bien une prothèse intraprostatique, ou bien une prothèse destinée à l'urètre pénien dans son ensemble ou seulement à certaines zones de cet urètre pénien.

Dans la présente description et dans les revendications qui y sont annexées, afin de donner un sens à des qualifications telles que "amont", "aval", "ventral" ou "dorsal", on conviendra d'orienter la prothèse urétrale par rapport à l'urètre dans lequel elle est destinée à être introduite, en se référant au sens du flux urinaire. Dans ces conditions, la partie amont de la prothèse est celle la plus proche de la vessie et la face ventrale de cette prothèse est celle en contact avec la partie ventrale, c'est-à-dire inférieure, de la paroi interne de l'urètre. La partie aval de la prothèse est située à l'extrémité opposée de la partie amont. La face dorsale de la prothèse est la face opposée à la face ventrale par rapport à la ligne moyenne de ladite prothèse.

De façon générale, l'urètre peut être sujet à des rétrécissements soit au niveau de la prostate, par hypertrophie des adénomes prostatiques, soit au niveau de l'urètre pénien, la restriction de ce conduit générant la quasi totalité des problèmes rencontrés, avec notamment infections et douleurs associées. Pour remédier à un rétrécissement urétral, on a déjà proposé, dans le brevet européen 274 846, d'utiliser une prothèse de type grille, mais ce type de prothèse présente l'inconvénient de provoquer au cours du temps des tuméfactions du conduit urétral par bourgeonnement tissulaire dû à l'enrobage des fils de la grille, d'où il résulte des lésions importantes lorsqu'il s'avère nécessaire d'enlever la prothèse.

On a donc proposé, dans le brevet FR-A-2 667 783, de constituer une prothèse urétrale au moyen d'un tube souple, dont le diamètre initial extérieur est supérieur au diamètre interne de la zone d'urètre concernée et dont une bande longitudinale est retirée en définissant une ouverture longitudinale entre deux bords longitudinaux, le tube ainsi ouvert étant mis en place dans l'urètre avec ses deux bords longitudinaux rapprochés pour ramener le diamètre externe du tube à être sensiblement égal au diamètre interne de la zone d'urètre concernée, ledit tube étant maintenu dans l'urètre en raison du fait qu'élastiquement, il a une tendance naturelle à se rouvrir de part et d'autre de la ligne moyenne de son ouverture longitudinale. Dans la position d'utilisation d'une telle prothèse, les bords longitudinaux de l'ouverture longitudinale sont disposés vers la zone ventrale de l'urètre, qui est la zone la plus sensible. Les bords longitudinaux précités ayant tendance à s'écarter pour faire reprendre au tube sa forme naturelle, on assure, de ce fait, une communication entre l'intérieur du tube de prothèse et l'urètre en vue de l'évacuation des sécrétions de celui-ci. Le meilleur choix de l'épaisseur du tube résulte du compromis entre une petite épaisseur, laquelle permet un plus grand diamètre interne du tube favorisant le passage du flux urinaire, et une grande épaisseur, laquelle assure une forte tendance du tube à reprendre sa forme initiale en raison de son élasticité propre.

Dans la demande de brevet WO-93/20779, on a aussi proposé de munir une prothèse du type défini dans FR-A-2 667 783 précité, d'une gaine de guidage ménagée le long des bords de l'ouverture longitudinale du tube, cette gaine étant constituée par une pluralité de tronçons disposés en quinconce et permettant, intérieurement, le coulissement d'un fil-guide. On peut ainsi, avec le fil-guide, maintenir la prothèse dans une configuration où son ouverture longitudinale est fermée, ce qui permet sa mise en place aisée dans l'urètre. Cependant, les tronçons de gaine de guidage forment des reliefs le long des bords de l'ouverture longitudinale de la prothèse pour permettre auxdits bords de venir l'un contre l'autre en vue d'assurer le diamètre minimum de la prothèse au moment de sa mise en place ; il en résulte, pour le patient, un certain inconfort, notamment en position assise, même si les tronçons de gaine sont en appui sur la face dorsale de l'urètre. Par ailleurs, ces tronçons de gaine constituent une gêne pour la mise en place de la prothèse par simple coulissement dans l'urètre et il n'est pas possible d'utiliser, comme applicateur, un tubage externe pour faciliter la mise en place de la prothèse dans l'urètre en raison de l'encombrement des tronçons de gaine et de l'épaisseur minimum du tubage-applicateur, qui réduiraient d'autant le diamètre de la prothèse elle-même. On a donc proposé, dans le brevet DE-4 141 572, de remplacer les tronçons de gaine précités par des éléments d'agrafage en quinconce constitués d'une succession de pattes et de créneaux, les pattes de l'un des bords pouvant venir se loger dans les créneaux de l'autre bord et réciproquement, les pattes étant disposées dans le prolongement de la paroi de la prothèse et ne formant aucun relief par rapport à la surface extérieure de l'élément tubulaire dans sa configuration de mise en place.

Pour toutes les prothèses du type défini dans les brevets FR-A-2 667 783, WO-93/20779 et DE-4 141 572 précités, l'essentiel est que les bords longitudinaux de l'ouverture longitudinale du tube souple que constitue la prothèse s'écartent avec une force suffisante pour générer élastiquement une augmentation du diamètre de l'urètre d'où résultera une augmentation de la section de passage disponible pour l'urine dans la prothèse. On a déjà proposé, par exemple dans DE-4 141 572, de renforcer l'élasticité propre de la paroi du tube en y insérant des secteurs métalliques noyés dans l'épaisseur de ladite paroi ; malheureusement cette technique est très onéreuse et conduit, généralement, à une augmentation de l'épaisseur de la paroi de la prothèse d'où il résulte une diminution de la section de passage définie à l'intérieur de la prothèse.

Un des buts de l'invention est de proposer une prothèse urétrale, qui ne subit aucun déplacement ultérieur après mise en place et qui tend à restituer élastiquement les caractéristiques dimensionnelles normales du passage de l'urine.

Un autre but de l'invention est de proposer une prothèse urétrale susceptible d'être positionnée exactement au niveau choisi à savoir, la prostate, l'urètre bulbaire, l'urètre pénien, ou parfois encore les trois successivement. On avait déjà proposé, dans WO-93/20779 précité, de résoudre la difficulté de mise en place d'une prothèse urétrale par simple poussage de la prothèse dans l'urètre ; mais cette technique interdisait tout retour de positionnement, ce qui constituait une difficulté lorsque la prothèse était coulissée trop loin dans l'urètre : en effet, aucun tubage-applicateur ne pouvait être utilisé avec une prothèse de ce type, comme il a été ci-dessus expliqué.

Selon l'invention, on propose d'améliorer la force d'ouverture élastique de la prothèse, c'est-à-dire la force qui tend à l'écartement des bords longitudinaux du tube fendu, qui constitue la prothèse, sans y insérer un arc métallique.

La présente invention a, en conséquence, pour objet, une prothèse urétrale constituée par un élément souple réalisé en un matériau élastique biologiquement acceptable, ayant une longueur sensiblement égale à celle de la partie d'urètre à traiter, ledit élément constituant, lorsqu'il est en place dans l'urètre, une gouttière ayant une ouverture longitudinale entre deux bords longitudinaux mais étant mis en place dans l'urètre avec ses deux bords longitudinaux rapprochés pour constituer un tube dont le diamètre externe est voisin du diamètre interne de la zone d'urètre concernée, ladite gouttière étant maintenue en place dans l'urètre par la réaction élastique de sa paroi, qui, après mise en place dans l'urètre, tend à reprendre sa conformation d'origine en rétablissant l'ouverture longitudinale supprimée pour la mise en place, caractérisée par le fait qu'elle est constituée d'une feuille de matériau qui, avant sa conformation sous forme de tube cylindrique pour la mise en place dans l'urètre, a, à l'état libre, une courbure nulle ou inverse de celle qui lui est imposée pour constituer ledit tube.

En d'autres, termes, pour former le tube cylindrique introduit dans l'urètre, on prend ou bien une feuille plane en forme de rectangle allongé ou bien une gouttière, dont la concavité est inversée pour former le tube. On a constaté que l'on obtenait ainsi une grande force élastique s'exerçant sur les parois de l'urètre, sans qu'il soit nécessaire de prévoir des inserts métalliques, d'où résultent une limitation du coût de la prothèse et une amélioration de la section de passage du flux urinaire.

Avantageusement, chaque bord longitudinal de l'ouverture longitudinale de la feuille de matériau constitutive de la prothèse porte des éléments d'agrafage disposés en quinconce et percés, qui, lorsque les bords longitudinaux viennent en contact au moment de la mise en place de la prothèse, permettent le passage d'un fil longitudinal dans lesdits éléments d'agrafage pour maintenir lesdits bords longitudinaux en contact ; les éléments d'agrafage en quinconce sont, de préférence, constitués d'une succession de pattes et de créneaux, les pattes de l'un des bords pouvant venir se loger dans les créneaux de l'autre bord et réciproquement, les pattes étant disposées dans le prolongement de la paroi de la feuille constitutive de la prothèse et ne formant aucun relief par rapport à la surface extérieure de la configuration tubulaire de mise en place.

De préférence, la prothèse selon l'invention présente des extrémités transversales arrondies. On peut aussi prévoir que les angles des pattes des bords longitudinaux soient arrondis.

La présente invention a également pour objet un applicateur destiné à la mise en place d'une prothèse telle que ci-dessus définie. Selon l'invention, un tel applicateur comporte un introducteur constituant un fourreau cylindrique à l'intérieur duquel est susceptible de coulisser un piston tubulaire cylindrique, ledit piston comportant intérieurement des moyens de guidage permettant la mise en place d'un conduit optique, une circulation d'eau étant assurée dans le piston autour dudit conduit optique, l'ouverture aval du piston étant obturée par un joint d'étanchéité qui entoure le conduit optique, un logement étant ménagé, dans le prolongement de la paroi du piston, entre l'extrémité de l'introducteur et l'extrémité du piston lorsque ce dernier est placé dans sa position aval par rapport à l'introducteur, logement à l'intérieur duquel est disposée la prothèse selon l'invention conformée tubulairement de façon à présenter un diamètre extérieur sensiblement égal au diamètre intérieur de l'introducteur.

Dans un premier mode de réalisation, les moyens de guidage du conduit optique sont des nervures longitudinales orientées radialement vers l'axe du piston, la circulation d'eau étant assurée dans le piston dans les zones comprises entre les nervures : on peut prévoir que dans sa partie aval, le piston comporte une arrivée d'eau située un peu en amont du joint d'étanchéité du piston, par exemple un peu en aval des nervures. Selon un autre mode de réalisation, les moyens de guidage du conduit optique forment un manchon ayant un axe parallèle à celui du piston, ce manchon étant raccordé audit piston sur une zone de la face interne dudit piston, le joint d'étanchéité du piston étant constitué par un élément annulaire obturant l'espace libre existant entre le manchon et la face interne du piston, en aval de l'arrivée d'eau assurant la circulation d'eau dans le piston.

Avantageusement, l'introducteur et son piston coulissant comportent chacun une poignée de préhension, l'action simultanée sur les deux poignées permettant d'assurer le coulissement relatif de l'introducteur par rapport au piston ; le déplacement relatif précité peut être repéré extérieurement au moyen d'un dispositif de repérage, qui reste visible sur la partie de l'introducteur situé de façon permanente à l'extérieur de l'urètre ; un tel dispositif de repérage peut consister en la combinaison d'une fente longitudinale pratiquée dans la paroi de l'introducteur avec un ergot porté par le piston, le positionnement de l'ergot dans la fente permettant le repérage désiré ; la fente de l'introducteur peut être crantée, chaque cran correspondant à une longueur de prothèse à mettre en place dans le logement de l'extrémité amont de l'introduteur. L'extrémité amont de l'introducteur peut être biseautée pour faciliter son insertion dans l'urètre, le plan moyen de cette extrémité étant oblique par rapport à l'axe de l'introducteur.

On préfère, lorsque la prothèse comporte des éléments d'agrafage coopérant avec un fil d'agrafage, que ce dernier s'étende à l'intérieur du piston et ressorte à l'extérieur de celui-ci par la partie aval du piston.

Pour mieux faire comprendre l'objet de l'invention, on va en décrire maintenant, à titre d'exemple purement illustratif et non limitatif, un mode de réalisation représenté sur le dessin annexé.

Sur ce dessin :
- la figure 1 représente en coupe axiale (avec parties intermédiaires supprimées), un applicateur selon l'invention dans la position correspondant à l'introduction d'une prothèse dans l'urètre d'un patient ;
- la figure 2 représente une coupe selon II-II de la figure 1 ;
- la figure 3 représente, en perspective, une prothèse selon l'invention dans la conformation tubulaire correspondant à sa mise en place ;
- les figures 4a, 4b, 4c représentent, en perspective, trois conformations de prothèse selon l'invention à l'état libre, l'axe de la conformation tubulaire telle que représentée sur la figure 3 étant figuré en traits mixtes ;
- la figure 5 représente, en perspective, une vue selon V-V de la figure 1.

En se référant au dessin, on voit que l'on a désigné par 14 dans son ensemble une prothèse selon l'invention.

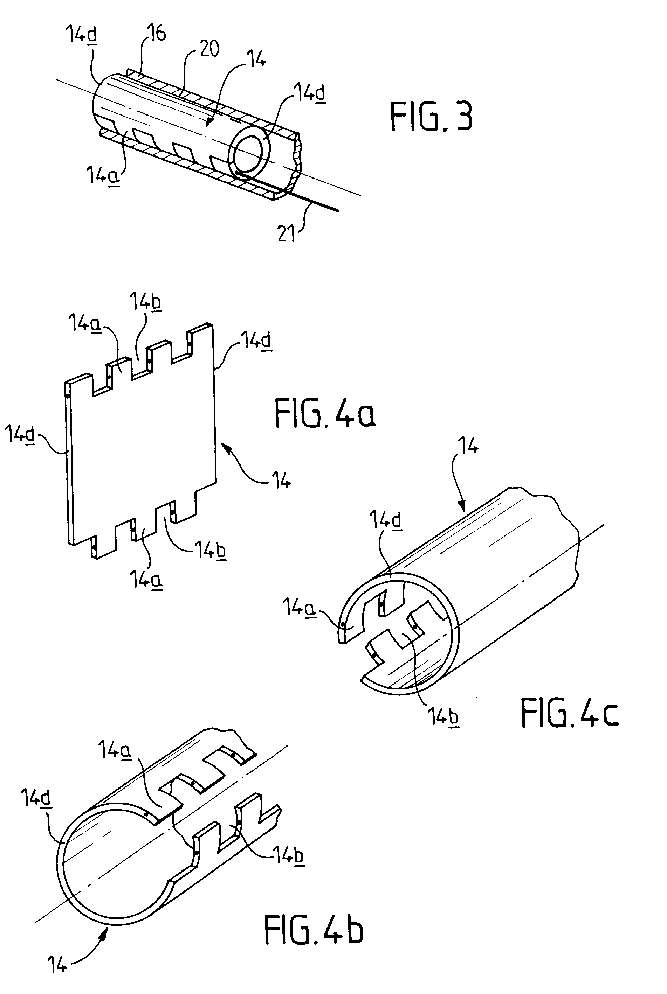

La prothèse 14 est constituée en un matériau plastique biocompatible ; à l'état libre elle peut avoir l'une des formes représentées sur les figures 4a à 4c, c'est-à-dire qu'elle peut être constituée par une paroi plane ou par une gouttière formant un secteur cylindrique. Dans tous les cas, la prothèse 14 est destinée à être conformée pour sa mise en place sous une forme tubulaire comme représenté sur la figure 3. L'axe de la conformation tubulaire est représenté sur les figures 4a à 4c en traits mixtes par rapport à la paroi de la prothèse à l'état libre ; la gouttière est dite concave lorsque l'axe de la conformation tubulaire finale est dans la concavité de la gouttière ; elle est dite convexe dans le cas contraire.

La prothèse 14 comporte sur chacun de ses bords longitudinaux une succession de pattes 14a et de créneaux 14b de forme sensiblement rectangulaire destinés, lorsque les deux bords longitudinaux de la prothèse sont rapprochés pour obtenir la conformation tubulaire de mise en place représentée sur la figure 3, à constituer un assemblage mâle-femelle, les pattes 14a d'un bord s'emboîtant dans les créneaux 14b de l'autre bord. Dans l'épaisseur de la paroi constitutive de la prothèse 14 et parallèlement à ses bords longitudinaux, on a ménagé dans les pattes 14a des percements 14c, qui, lorsque les deux bords longitudinaux de la prothèse sont rapprochés et assemblés avec emboîtage des pattes 14a dans les créneaux 14b, sont alignés pour permettre le passage d'un fil de maintien ou d'agrafage 21. Le fil 21 maintient la paroi constitutive de la prothèse dans une conformation cylindrique représentée sur la figure 3, conformation dont le diamètre extérieur correspond au diamètre intérieur du logement 20, qui sera défini ci-après. Le fil 21 est destiné à être retiré quand la prothèse a été mise en place à l'endroit désiré dans l'urètre, de façon que la paroi de la prothèse 14 tende élastiquement à revenir à la forme qu'elle avait à l'état libre c'est-à-dire à l'une des formes représentées sur les figures 4a à 4c. Dans le cas d'une forme initiale en gouttière convexe (figure 4c), la concavité de la paroi de la prothèse a été inversée pour la mise en place du fil 21 et la force élastique de retour à la forme initiale est plus grande que dans le cas d'une gouttière concave (figure 4b) ; le cas de la paroi initialement plane (figure 4a) est intermédiaire entre le cas d'une gouttière concave et le cas d'une gouttière convexe. Cette force élastique tendant au retour à la forme initiale assure le maintien de la prothèse dans l'urètre et l'ouverture optimum de la section droite de la zone d'urêtre ou s'effectue la mise en place de la prothèse.

La prothèse 14 présente, en outre, des extrémités transversales 14d qui sont dans des plans sensiblement perpendiculaires aux bords longitudinaux crénelés ; ces extrémités 14d sont arrondies, l'arrondi permettant une mise en place aisée de la prothèse sans blesser le conduit urétral. La longueur de la prothèse est généralement de l'ordre de 2 à 4 centimètres selon la zone d'urètre à traiter ; son diamètre, quand elle est mise dans sa conformation tubulaire, permet une implantation dans toutes les zones de l'urètre sachant que l'ouverture élastique de la prothèse in situ rattrape les variations de diamètre de l'urètre.

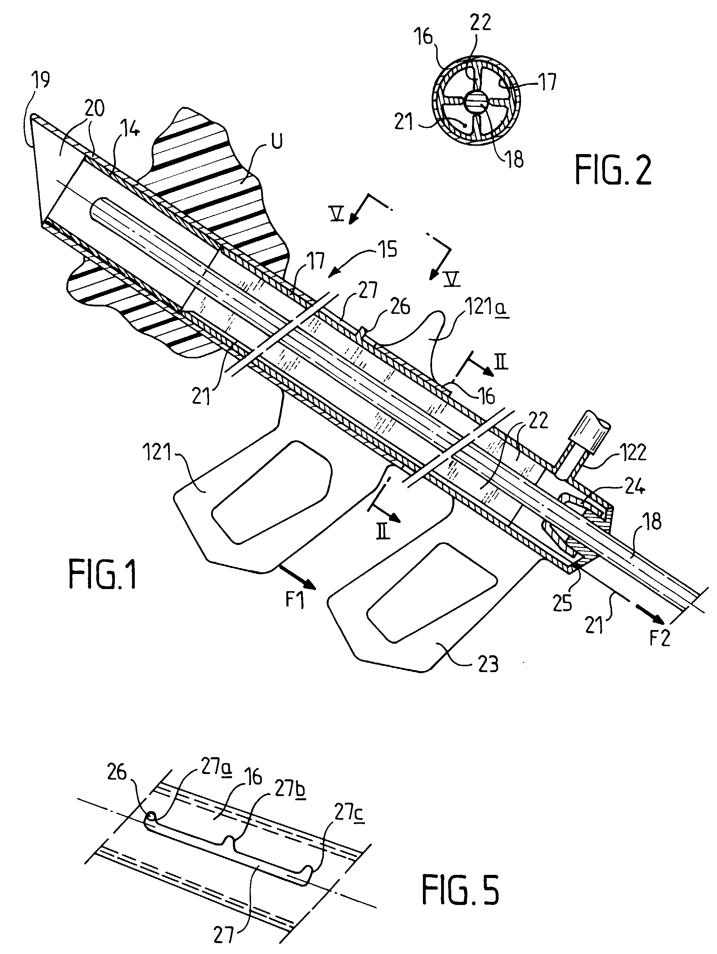

L'applicateur de prothèse selon l'invention est désigné par 15 dans son ensemble sur la figure 1 ; il comporte essentiellement un introducteur 16 et un piston d'application 17 portant en glissement interne un conduit optique 18 ; un logement 20 de la prothèse 14 est ménagé en amont à l'intérieur dudit introducteur 16, dans le prolongement du piston 17. Le piston 17 est susceptible de coulisser à l'intérieur de l'introducteur 16 ; il comporte intérieurement des nervures 22, qui guident entre elles la mise en place par coulissement du conduit optique 18.

L'introducteur 16 constitue une tubulure cylindrique, qui est biseautée en forme de sifflet 19 à son extrémité amont afin de faciliter son introduction dans l'urètre. L'introduteur 16 constitue un fourreau cylindrique, qui peut être métallique, par exemple en titane ou en un alliage similaire de faible épaisseur ; il peut porter une poignée de préhension 121 associée, du côté diamétralement opposé de l'introducteur, à un doigt d'appui 121a.

Le piston 17 est également un cylindre mince qui peut aussi être métallique ; il comporte intérieurement des nervures axiales 22, rectilignes ou non, par exemple, disposées radialement au nombre de quatre comme il est visible sur la figure 2. Ces nervures guident et maintiennent le conduit optique 18 et permettent sa mise en place par glissement axial à l'intérieur de l'introducteur. Le piston 17 comporte extérieurement une arrivée d'eau 122 et une poignée de préhension 23. Un joint d'étanchéité 24 est disposé entre, d'une part, l'extrémité aval du piston 17 qui est opposé à celle adjacente au logement 20, et d'autre part, la paroi externe du conduit optique 18, pour limiter autant que possible les fuites d'eau.

On dispose la prothèse 14, mise dans sa conformation tubulaire grâce au fil d'agrafage 21, dans son logement 20, de façon que le fil 21 se place dans un compartiment entre deux nervures adjacentes 22 comme il est visible sur les figures 1 et 2 ; le fil 21 émerge du piston par un orifice 25 où l'on peut également prévoir un joint d'étanchéité. De la sorte, il est possible pour le praticien urologue de tirer sur le fil 21 comme il sera explicité ci-après.

Enfin, on a prévu sur la paroi latérale de l'introducteur une fente longitudinale crantée 27 à l'intérieur de laquelle se trouve un ergot 26 qui est lié au piston 17. L'ergot 26 peut être positionné dans l'un des crans 27a 27b 27c répartis sur la longueur de la fente 27 par une rotation du piston 17 par rapport à l'introducteur 16. Ceci permet de positionner la prothèse 14 dans son logement 20 en fonction de sa longueur. Bien entendu, il serait possible de mettre en place de simples repères dimensionnels le long de la fente 27 pour obtenir le même résultat. Lorsque l'on veut permettre le coulissement relatif de l'introducteur 16 par rapport au piston 17, il convient, bien entendu, de sortir préalablement l'ergot 26 du cran latéral où il a été positionné de façon à le ramener dans l'axe de la fente 27.

Pour procéder à la mise en place de la prothèse urétrale selon l'invention, on amène la prothèse 14 dans sa conformation tubulaire où les pattes 14a sont positionnées dans les créneaux 14b et on fixe la prothèse dans cette conformation au moyen du fil d'agrafage 21. On met ensuite en place la prothèse 14 dans le logement 20 de l'applicateur 15, le fil 21 dépassant largement vers l'extérieur par l'orifice 25 ; en variant on peut prévoir de disposer le fil 21 à l'intérieur d'une fente longitudinale pratiquée dans l'épaisseur de la paroi de l'introducteur 16. On introduit ensuite dans l'urètre l'extrémité biseautée 19 de l'introducteur 16, l'urologue s'aidant pour cette introduction du conduit optique 18 et des moyens radioscopiques usuels jusqu'à ce que l'on atteigne pour la prothèse 14 la position souhaitée qui peut être dans la région péniène, bulbaire ou prostatique de l'urètre. L'urologue peut ajuster très exactement le positionnement de la prothèse en poussant ou tirant l'introducteur 16 ; quand la localisation désirée a été atteinte de façon précise, le praticien tire vers l'aval, selon la flèche F1, l'applicateur 16 en maintenant sensiblement en position fixe le piston 17 ; de la sorte la prothèse 14 est extraite de son logement 20 ; on tire alors sur le fil 21 selon la flèche F2 pour produire le désagrafage de la prothèse 14 dont la paroi, par élasticité, tend à reprendre sa conformation initiale en s'appuyant élastiquement contre la paroi de l'urètre. La prothèse ainsi mise en place peut, en fonction du conduit urétral, occuper une position plus ou moins ouverte, ses bords longitudinaux étant plus ou moins écartés l'un de l'autre ; au cours du temps la prothèse occupe en raison de son élasticité propre une position optimale qui permet de réaliser dans la zone d'urètre traitée une section maximale pour le passage de l'urine tout en assurant une position stable dans l'urètre, une bonne tolérance par le patient. En outre il est possible sans trop de difficulté d'éliminer ultérieurement la prothèse. On constate donc que la prothèse selon l'invention peut être mise en place de façon très précise grâce à l'applicateur qui a été ci-dessus décrit.

## Revendications

1. Prothèse urétrale (14) constituée par un élément souple réalisé en un matériau élastique biologiquement acceptable, ayant une longueur sensiblement égale à celle de la partie d'urètre à traiter, ledit élément constituant, lorsqu'il est en place dans l'urètre (U), une gouttière ayant une ouverture longitudinale entre deux bords longitudinaux mais étant mis en place dans l'urètre (U) avec ses deux bords longitudinaux rapprochés pour constituer un tube dont le diamètre externe est voisin du diamètre interne de la zone d'urètre concernée, ladite gouttière étant maintenue en place dans l'urètre (U) par la réaction élastique propre de sa paroi, qui, après mise en place dans l'urètre, tend à reprendre sa conformation d'origine en rétablissant l'ouverture longitudinale supprimée pour la mise en place, caractérisée par le fait qu'elle est constituée d'une feuille de matériau qui, avant sa conformation sous forme de tube cylindrique pour la mise en place dans l'urètre, a, à l'état libre, une courbure nulle ou inverse de celle qui lui est imposée pour constituer ledit tube.

2. Prothèse selon la revendication 1, caractérisée par le fait que chaque bord longitudinal de l'ouverture longitudinale de la feuille de matériau constitutive de la prothèse porte des éléments d'agrafage disposés en quinconce et percés, qui, lorsque les bords longitudinaux viennent en contact au moment de la mise en place de la prothèse, permettent le passage d'un fil longitudinal dans lesdits éléments d'agrafage pour maintenir lesdits bords longitudinaux en contact.

3. Prothèse selon la revendication 2, caractérisée par le fait que les éléments d'agrafage en quinconce sont constitués d'une succession de pattes (14a) et de créneaux (14b), les pattes (14a) de l'un des bords pouvant venir se loger dans les créneaux (14b) de l'autre bord et réciproquement, les pattes (14a) étant disposées dans le prolongement de la paroi de la prothèse (14) et ne formant aucun relief par rapport à la surface extérieure de l'élément tubulaire dans sa configuration de mise en place.

4. Prothèse selon l'une des revendications 1 à 3, caractérisée par le fait que ses extrémités transversales (14d) sont arrondies.

5. Applicateur destiné à la mise en place d'une prothèse selon l'une des revendications 1 à 4, caractérisé par le fait qu'il comporte un introducteur (16) constituant un fourreau cylindrique à l'intérieur duquel est susceptible de coulisser un piston (17) tubulaire cyclindrique, ledit piston (17) comportant intérieurement des moyens de guidage (22) permettant la mise en place d'un conduit optique (18), une circulation d'eau étant assurée dans le piston (17) autour dudit conduit optique (18), l'ouverture aval du piston (17) étant obturée par un joint d'étanchéité (24), qui entoure le conduit optique (18), un logement (20) étant ménagé entre l'extrémité de l'introducteur (16) et l'extrémité du piston (17) lorsque ce dernier est placé dans sa position aval, logement à l'intérieur duquel est disposée la prothèse (14) conformée tubulairement.

6. Applicateur selon la revendication 5, caractérisé par le fait que les moyens de guidage du conduit optique (18) sont des nervures longitudinales (22) orientées radialement vers l'axe du piston (17), la circulation d'eau étant assurée, dans le piston (17), dans les zones comprises entre les nervures (22).

7. Applicateur selon la revendication 6, caractérisé par le fait que, dans sa partie aval, le piston comporte une arrivée d'eau (122) située un peu en amont du joint d'étanchéité (24) du piston (17).

8. Applicateur selon la revendication 5, caractérisé par le fait que les moyens de guidage du conduit optique (18) forment un manchon ayant un axe parallèle à celui du piston (17), ce manchon étant raccordé audit piston sur une zone de la face interne dudit piston, le joint d'étanchéité du piston étant constitué par un élément annulaire obturant l'espace libre existant entre le manchon et la face interne du piston (17), en aval de l'arrivée d'eau assurant la circulation d'eau dans le piston.

9. Applicateur selon l'une des revendications 5 à 8, caractérisé par le fait que l'introducteur (16) et son piston coulissant (17) comportent chacun une poignée de préhension (121, 23), l'action simultanée sur les deux poignées permettant d'assurer le coulissement relatif de l'introducteur (16) par rapport au piston (17).

10. Applicateur selon l'une des revendications 6 à 9, caractérisé par le fait que le déplacement relatif de l'introducteur (16) par rapport au piston (17) est repéré extérieurement au moyen d'un dispositif de repérage visible sur la partie de l'introducteur (16) située de façon permanente à l'extérieur de l'urètre (U).

11. Applicateur selon la revendication 10, caractérisé par le fait que le dispositif de repérage consiste en la combinaison d'une fente (27) longitudinale pratiquée dans la paroi de l'introducteur (16) avec un ergot (26) porté par le piston.

12. Applicateur selon la revendication 11, caractérisé par le fait que la fente comporte des crans latéraux dans lesquels peut pénétrer l'ergot (26) du piston (17) par une rotation du piston par rapport à l'introducteur (16).

13. Applicateur selon l'une des revendications 5 à 12, utilisable pour la mise en place d'une prothèse selon l'une des revendications 2 ou 3, caractérisé par le fait que le fil (21) d'agrafage de la prothèse (14) s'étend à l'intérieur du piston (17) et ressort à l'extérieur de celui-ci par la partie aval du piston (17).
